# EUROPEAN PATENT APPLICATION

(11) **EP 2 745 688 A1**
(43) Date of publication of application: **25.06.2014**
(21) Application number: 13198116.9
(22) Date of filing: 18.12.2013
(51) Int. Cl.: A01M 1/24, A01K 31/14

(54) **Built-in housing for nurturing or controlling creatures**

(30) Priority: 18.12.2012 GB 201222877
(71) Applicant: McCutchan, Duncan Benedict, Hailsham East Sussex BN27 1EP (GB)
(72) Inventor: McCutchan, Duncan Benedict, Hailsham East Sussex BN27 1EP (GB)
(74) Representative: Giles, Ashley Simon

(57) **Abstract**

Apparatus for nurturing or controlling creatures, the apparatus comprising a housing adapted to be built in to a structure, such as a wall floor or ceiling, and a plurality of removable and exchangeable inserts which are adapted to fit into the housing, each of the inserts having a different function.

## Description

This invention relates to a housing for building into a structure and particularly, although not exclusively, relates to a box for housing or controlling creatures which is built into a wall of a building.

### BACKGROUND

Conventional bird shelters generally comprise wooden boxes which are attached to the outside of a structure, or are placed on a stand. It is also known to mount a bird box in a wall. When the wall is built, the bird box is inserted into the wall in place of one or more blocks. An opening is provided in the front of the box to allow access. A modified version of this box can be used to provide shelter for bats. In this case, a slot is provided in the front of the box to provide an access opening which is suitable for bats. GB2479004 discloses a housing of this kind which is built into a wall.

### STATEMENTS OF INVENTION

According to a first aspect of the present invention there is provided apparatus for nurturing or controlling creatures, the apparatus comprising a housing adapted to be built in to a structure, such as a wall floor or ceiling, and a plurality of removable and exchangeable inserts which are adapted to fit into the housing.

The housing may comprise a box for housing or controlling creatures. In the present context the term "controlling" creatures means killing, causing to be killed, sterilizing or otherwise reducing the population of the creatures.

The inserts may comprises a bait station, a trap or a habitat enhancer. At least one of the inserts may be adapted to have a trap mounted on it.
The habitat enhancer may customises the housing for a particular bird, animal or insect. For example, the habitat enhancer may comprise a hive support for adapting the housing for use by bees or other hive insects. Alternatively, the habitat enhancer may comprise a nest support frame for assisting birds in forming a nest.

The housing may comprise a container portion and a lid. The lid may be provided with a peripheral flange which accommodates a decorative covering, the peripheral flange being closely received within the container portion. A free edge of the container portion may comprise a mortar barrier which separates mortar on the lid from mortar applied to the wall. At least one of the inserts may be connected to the lid. At least one of the inserts is may be removably connected to the lid. At least one of the inserts may be permanently connected to the lid. The lid may be hinged to the container portion. Th lid and at least one of the inserts may be provided with cooperating formations, such as barbed tangs and cooperating openings.The barbed tangs may project from an inside face of the lid and the cooperating openings may be formed in the insert.

The inserts supplied with the housing may be functionally different from one another. For example, one may be for nurturing birds, another may be for nurturing bees, another may be for killing rodents and another may be for killing cockroaches.

The housing may be adapted to accommodate a plurality of inserts at the same time. For example, with appropriate inserts it could operate as a combined rodent and cockroach trap.

According to another aspect of the present invention, there is provided a built-in housing for controlling or nurturing creatures, the housing comprising a container portion, a lid and an insert attached to the lid, the insert comprising a habitat enhancer, a bait station or a trap, the lid and insert being removable together as a unit.

According to another aspect of the present invention there is provided a housing for building into a structure, the housing comprising a container portion and a lid adapted to support a decorative covering which is attached to the lid by adhesive, the lid being provided with a plurality of openings which are size to allow a predetermined amount of the adhesive to seep into the openings as it sets.

One or more of the openings may comprise perforations. The perforations may be sized to allow a predetermined amount of the adhesive to seep through the openings as it sets. One or more of the openings may be of circular cross section. One or more of the openings may be elongate. One or more of the openings may be cross shaped (cruciate), L-shaped, T-shaped or polygonal. One or more of the openings may taper.

The decorative covering may comprise slices of block. The slices of block may be formed from the same blocks as the blocks of the wall. The slices of block may be formed from actual blocks of the wall.

The lid may be cross shaped (cruciate) or L-shaped or T-shaped when viewed in the plane of a wall to which it is fitted.

The lid may be mounted in a wall, such that an exposed face of the decorative covering is substantially flush with the wall.

The container portion may be provided with sockets which are adapted to receive fixings which pass through or extend from the lid.

The lid may be provided with a peripheral flange which accommodates the decorative covering. The peripheral flange may be closely received within the container portion.

In the assembled housing, the free edge of the flange may be substantially flush with a free edge of the container portion.

At least a part of an outer surface of the peripheral flange may taper outwardly.

At least a part of an inner surface of a side wall of the container portion may taper outwardly towards a mouth of the container portion.

The side wall of the container portion adjacent the mouth may be adapted to act as a mortar barrier which separates the cover from mortar applied to the wall and prevents unwanted adhesion of the mortar to the lid.

If the housing is adapted to replace a plurality of blocks of a wall, it is more spacious internally than a conventional housing which replaces a single block of a wall. Consequently, a housing in accordance with the present invention can accommodate larger birds, insects or animals. Alternatively, it could accommodate a plurality of birds, insects or animals. For example, a single container could house a roost of bats. It could also accommodate a camera for observing a bird, animal or insect or for observation or surveillance more generally inside or outside the container.

In addition, a housing in accordance with the present invention which replaces a plurality of blocks of a wall, will look more visually striking , if it is made from a different material or in a different colour from the other blocks of the wall. In particular, if the housing has a substantially cruciate shape, it will look particularly striking on an otherwise blank wall.

In an embodiment, the container portion may have an open mouth, which receives the cover, the container portion being mounted in the wall such that the mouth is substantially flush with a front face of the wall and the cover comprises a raised lip, the cover being adapted to be closely received in the mouth of the container portion.

According to another aspect of the present invention there is provided a housing for building into a structure, the housing comprising a container portion and a lid, the container portion having an open mouth defined by a peripheral lip and the lid having a raised peripheral flange which is configured to be closely received in the lip of the container portion. The housing may comprise a box for housing or controlling creatures and/or the structure may comprise a wall floor or ceiling of a building.

An outer surface of the flange may taper outwardly towards a free edge of the flange.

At least a part of the inner surface of the container portion may taper outwardly towards the open mouth.

The lip may act as a mortar barrier. The mortar barrier separates mortar on the cover from mortar applied to the wall.

The lip may be formed from less thick material than the rest of the container portion.

In the above described aspects of the invention the housing may further comprise a removable insert.

According to another aspect of the present invention there is provided a housing for building into a structure, the housing comprising a container portion, a lid and a removable insert.

The housing may comprise a box for housing or controlling creatures and/or the structure may comprise a wall floor or ceiling of a building.

The insert may comprise a habitat enhancer, a bait station or a trap. If the insert comprises a habitat enhancer, the insert customizes the housing for a particular bird, animal or insect. For example, the habitat enhancer may comprise a hive support for adapting the housing for use by bees or other hive insects, which can form their hive on the insert. In an alternative embodiment, the insert may comprise a panel, such as a wooden panel, which can be gripped easily by bats when they roost.

The insert may comprise at least one support element which may hold the insert in a substantially vertically aligned orientation in the container portion.

The insert may be attached to the lid. The lid may be removable.

According to another aspect of the present invention there is provided a kit of parts comprising a housing as described above and a plurality of removable and exchangeable inserts.

The housing may comprise a container portion adapted to receive a lid. At least one of the inserts may be permanently or removably attached to the lid. In one embodiment the insert and lid together form a replaceable or serviceable unit, so that by removing the lid, the insert is also removed from the container portion for servicing or replacement by a fresh or alternative unit.

After a period of use, the lid and insert may be removed together, so that an operator only has to touch the lid to remove the insert. This is particularly advantageous where the insert is a bait station or trap, since the operator does not need to touch the insert during removal from the container portion. The lid and insert can then be disposed of together and a fresh lid and insert placed into the container portion as a unit, or the unit can be cleaned and replenished with poison bait, or the trap can be reset for further use.

With the insert attached to the lid, the insert can be inserted or removed in a hygienic manner, with the operator only needing to touch the lid. Therefore the housing is suitable for sale to domestic as well as commercial properties and can be serviced by less skilled operatives, or by the property owner themselves.

If it is appropriate to adapt the housing for a different use, the lid and insert can be removed from the container portion and exchanged with an alternative lid and/or insert. In an alternative embodiment, the insert can be disconnected from the lid and an alternative insert can be fixed to the same lid. Any means of fixing the lid to the or each insert is contemplated. For example, the insert may be clipped onto the lid by means of inter-engaging formations such as barbs moulded into the lid which engage in cooperating openings formed in the or each insert, or the lid and insert may be screwed or bolted together.

If the insert is removably connected to the lid, the housing could be provided with alternative lids which are each adapted to fit to a wall, floor or ceiling with a different surface appearance. For example, one of the lids may have a smooth surface finish, another may have a roughened finish to match a rendered surface, one may comprise a simulated brick or block pattern, and one may comprise real slices of brick or block. The colour of the lids may also be varied to suit specific applications. If the container portion and/or the lid, and/or the insert are formed from plastics material, the colour may be moulded into the material. Alternatively, the colour may be applied as a coating, treatment, or paint.

According to another aspect of the present invention, there is provided a wall, floor or ceiling mounted housing for controlling or nurturing creatures, the housing comprising a container portion, a lid and an insert attached to the lid, the insert comprising a habitat enhancer, a bait station or a trap, the lid and insert being removable together as a unit.

If the insert comprises a habitat enhancer, the insert customizes the housing for a particular bird, animal or insect. The habitat enhancer may comprise a hive support for adapting the housing for use by bees or other hive insects, which can form their hive on the insert. Alternatively, the insert may comprise a panel, such as a wooden panel which can be gripped easily by bats when they roost.

According to another aspect of the present invention, there is provided a kit of parts forming an assembly which is mounted in a wall, floor or ceiling, the kit comprising a housing and a plurality of removable and exchangeable inserts which are adapted to fit into the housing. The housing may comprise a container portion and a lid. At least one of the inserts may be attached to the lid. Each of the inserts may be attached to a respective lid, and may therefore form a cartridge assembly which can be removed in one piece from the container portion.

The insert, or at least one of the inserts of the kit of parts, may comprise a habitat enhancer which customises the housing for a particular bird, animal or insect. For example, one insert may comprise a hive support for adapting the housing for use by bees or other hive insects. Another of the inserts may comprise a panel, such as a wooden panel, which can be gripped easily by bats when they roost. The wooden panel may be made from a piece of oak. Another of the inserts may comprise a nest support frame for assisting birds in forming a nest.

The insert, or one of the inserts of the kit of parts, may comprises a bait station or trap, so that the container portion lid and insert together form a pest control unit which can be built into a structure. The container portion may be built into a lower course of blocks of an interior or exterior wall. For example, if the container portion is built into the interior wall of a restaurant, and is provided with a trap or bait station it could be used for catching or killing rodents or insects such as cockroaches. In one embodiment, the insert comprises a cockroach trap in the form of a sticky pad. When the cockroach walks onto the pad it becomes stuck, and can be destroyed when the sticky trap is cleaned off or replaced, or the sticky pad may be impregnated with poison which kills the cockroach in situ.

The cover may be provided with an opening or may be sized to provide a gap between the cover and the container portion, so that creatures to be killed or trapped can access the container portion. In addition or instead, a wall of the container portion may be provided with an opening to allow creatures to enter the trap or bait box through a wall cavity or fissure in the wall. For example, if a rear wall of the container portion is provided with an opening, the container portion can be accessed by creatures which are present in the wall cavity. It will be appreciated that creatures such as rodents and cockroaches often seek out dark recesses in holes in walls or gaps in structures, so a built-in trap or bait box with an appropriate opening from the wall cavity would be very attractive to the creatures for which the trap or bait box is intended.

As the trap or bait box is hidden from view within the structure to which the container portion is fitted, it can be used in a hotel or restaurant or other public space without disturbing customers or other members of the public.

It can be off putting for a restaurant customer or hotel guest to see a bait box or trap in a restaurant or hotel, so being able to hide these essential structures from view, whilst enhancing their effectiveness is a very significant benefit of the present invention.

The lid may hinged to the container portion along a top edge, so that gravity alone keeps the lid shut, or it may be hinged along a side or bottom edge and provided with a mechanism to hold the lid closed. Alternatively, the lid may be screwed or clipped to the container portion, or may be otherwise made completely removable from the container portion for easy access to the container portion and for replacement or substitution of one insert with another of the same or a different type.

The insert may be adapted to be closely received in the container portion.

### BRIEF DESCRIPTION OF DRAWINGS

For a better understanding of the present invention and to show how it may be carried into effect, reference will now be made, by way of example, to the accompanying drawings in which:
Figure 1 shows a bird nesting box with the cover removed;
Figure 2 illustrates a first stage in the construction of a wall incorporating a housing in accordance with the present invention;
Figure 3 illustrates a second stage in construction of the wall of Figure 2;
Figure 4 illustrates a third stage in construction of the wall of Figure 2;
Figure 5 illustrates the final stage in construction of the wall of Figure 2;
Figure 6 is a perspective view of the container portion of the housing with the cover removed;
Figure 7 is a perspective view of the lid of the housing;
Figure 8A is a plan view of an embodiment of the lid having a perforated panel;
Figure 8B is a side view of the lid of Figure 8A;
Figure 8C is an end view of the lid of Figure 8A;
Figure 9A is a plan view of an embodiment of the lid having a wire mesh panel;
Figure 9B is a side view of the lid of Figure 9A;
Figure 9C is an end view of the lid of Figure 9A;
Figure 10 illustrates an insert for roosting bats being located in the container portion;
Figure 11 illustrates an alternative insert with side support bars being located in the container portion;
Figure 12 illustrates an alternative insert with both support feet and side support bars being located in the container portion;
Figure 13 illustrates an alternative insert comprising a combined rodent bait station and insect trap;
Figure 14 illustrates an alternative insert comprising an insect sticky trap;
Figure 15 illustrates an alternative insert comprising a rodent bait station;
Figure 16 illustrates an alternative insert comprising a rodent trap; and
Figure 17 illustrates an alternative insert comprising a rodent bait station with a reversible lid.

### DETAILED DESCRIPTION OF PREFERRED EMBODIMENTS

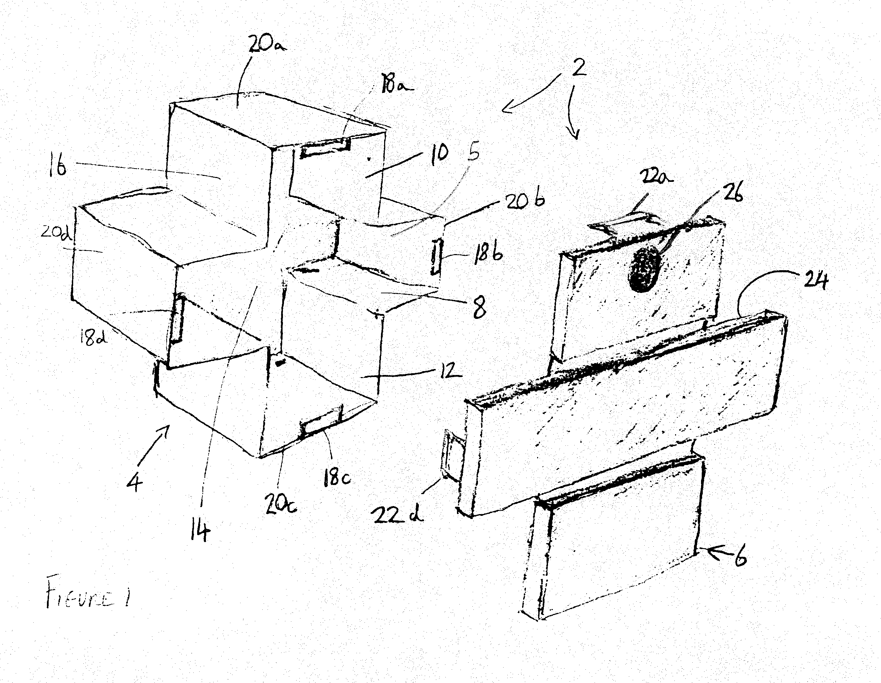

Referring to the drawings, Figure 1 illustrates a first embodiment of animal shelter comprising a bird nesting box 2 comprising a substantially cross-shaped housing 4 and a substantially cross-shaped cover or lid 6. The housing 4 defines a container portion 5 comprising a substantially horizontally aligned compartment 8 which is integrally formed with an upper compartment 10 and a lower compartment 12. A back wall 14 and peripheral wall 16 may be moulded together from plastics material to form the housing 4, and the cover 6 may also be moulded in one piece from plastics material. In alternative embodiments, the compartments of the bird nesting box may be formed from individually formed walls which may be wholly or partially pre-assembled prior to sale or may be supplied in kit form for assembly on site. Furthermore, the bird nesting box may be formed in whole or in part from plastics material, or any other suitably rigid material such as aluminium, galvanised steel, concrete or composite material such as fibreglass.

In the illustrated embodiment, tangs 18a, 18b, 18c and 18d are moulded onto the outer walls 20a, 20b, 20c and 20d of the housing 4 towards the front of the housing 4 and engage with corresponding retaining lugs 22a, 22b, 22c and 22d which are moulded into a face 24 of the cover 6 at the outer edges of the cover 6. An access opening 26 is formed through the cover 6. In the present embodiment, the access opening 26 is circular to allow easy access for birds and is situated towards the top of the cover. In alternative embodiments, the shape and/or size and/or position of the access opening 26 may be varied. For example it may comprise a slot to provide access for bats. The access opening 26 may be moulded into the cover 6 or may formed later. Where the access opening 26 is circular in cross section it may be drilled into the cover 6 using a conventional hole saw. Where the access opening 26 is a slot, it may be formed by sawing it out of the cover, for example with a hacksaw or jigsaw. Where the cover 6 is cross-shaped, the access opening could be formed in a lower edge of a side arm of the cover, so that it lines up with the horizontally aligned compartment 8. This arrangement has the advantage that if the access opening 26 is a slot, it is easy to form by making only two intersecting cuts from the bottom edge and side edge of the cover 6. Also, because the slot it not adjacent the lowermost part of the container portion 5, it will not become blocked with bat guano, even after many years of continuous use by bats.

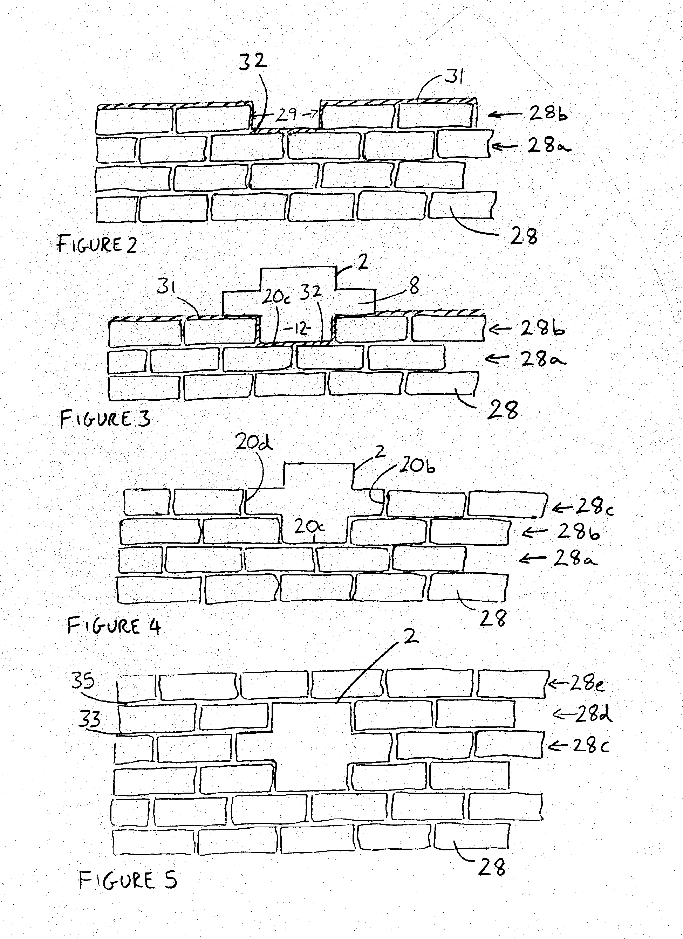

Referring to Figure 2, in use of the bird nesting box 2, a brick wall 28 is first constructed up to a course 28a at the proposed height of the lower wall 20c of the nesting box 2. A further course of bricks 28b is then laid, leaving a gap of sufficient size to accommodate the nesting box 2. In the embodiment illustrated, a gap 29 slightly wider than the compartment 12 is left in the course of bricks 28b. A bed of mortar 31 is laid along the top of the course of bricks 28b and a bed of mortar 32 is laid in the bottom of the gap.

Referring to Figure 3, the nesting box 2 is then bedded into the wall 28, such that the lower wall 20c of the nesting box 2 is laid onto the bed of mortar 32 on the course of bricks 28a and the substantially horizontally aligned compartment 8 sits on the bed of mortar 31 on the course of bricks 28b.

Referring to Figure 4, a further course of bricks 28c is then laid onto the course 28b, such that respective bricks of the course 28c abut the end walls 20b, 20d of the nesting box 2.

Referring to Figure 5, two more courses of bricks 28d, 28e are laid on respective beds of mortar 33, 35, such that the nesting box 2 is fully embedded in the wall 28. Further courses of bricks are added to complete the wall, and the wall is then pointed in a conventional manner. During the process of pointing the wall, the joints between the various brick courses and the nesting box 2 are also pointed.

It will be appreciated that by making the width of the housing 4 in the region of the upper and lower compartments 10, 12 equal to the width of a standard brick, and the width of the housing 4 in the region of the substantially horizontally aligned compartment 8 equal to the length of a standard brick, the bird box fits easily into the wall and an equal mortar gap is maintained around the bird box as is used in the rest of the wall 28.

Once the mortar has set, the cover 6 can be pushed into place onto the housing 4 such that the retaining lugs 28a, 28b, 28c and 28d on the cover 6 engage with the corresponding tangs 18a, 18b, 18c and 18d on the housing 4. In the illustrated embodiment, the lugs snap fit into position, but in alternative embodiments (not illustrated) they may simply engage as a friction fit with the tangs, or screws may be secured into the housing 4 through holes formed in the cover 6.

In order to improve the appearance of the bird box 2 in the wall 28, the cover 6 may be coloured or patterned. For example the cover may be moulded to reproduce the shape of the bricks in the wall. Alternatively, brick or part brick shaped elements may be attached to the cover either at the time of manufacture or on site. In one alternative embodiment, actual bricks from the wall may be cut to produce the sections which can be attached to the cover by screws, adhesive or any other suitable means to reproduce exactly the contour of the wall 28 over the visible surface of the bird box 2.

The access opening 26 is sized and shaped to allow access by birds which are to nest in the box 2. It will be appreciated that the invention could be adapted for other purposes. For example, as mentioned above, the access opening 26 could comprise a slot which provides access to bats rather than birds. Similarly, the invention could be adapted to provide accommodation for reptiles or insects either for the purpose of nurturing them or as a bait box into which traps or poisoned bait could be laid and into which only pests such as rats and mice can gain access. In this embodiment, the bait box may be laid into the wall substantially at ground level, or above or below the damp proof course. As the housing extends over an area greater than that of a single block, it provides a large reservoir for bait, or a large space which can accommodate more than one trap.

The invention may also be applied as a retrofit assembly. In this embodiment, a brick and two batts are removed from an existing wall and a nesting box 2 is cemented in their place. Generally, removing this many bricks will not weaken a wall to the extent that additional support is required, especially since a bat or bird nesting box is likely to be sited at the top of a wall close to the eaves, where loading is generally light. However, where additional support is required the housing 4 may be made of stiffer material or may be provided with internal bracing, provided that this does not unduly limit the useful space available in the box.

Alternatively, a lintel may be inserted above the box to support the wall above the box. This technique is used conventionally above door and window openings, so is not described in detail here.

The invention can be adapted to any structure, either externally or internally, where the structure is formed in whole or in part from blocks fitted together. For example it could be applied to any form of block work or brick work construction as well as to natural stone or even solid concrete walls simply by modifying the housing 4 and cover 6 to fit the pattern of the brick work, block work or stone work, or matching the cover 6 to the surface finish of a continuous wall such as a solid concrete wall. The box may be cast directly into a concrete wall either on site or at a pre-cast fabrication area.

In an alternative embodiment, the cover 6 may be deliberately contrasting in colour or contour to provide an attractive highlight in the wall.

An advantage of making the cover 6 removable is that access to the housing 4 is possible at any time, for example to clean out the old nest material or to displace unwanted creatures such as bees, wasps, hornets, or rodents or to install a camera to observe the movements and behaviour of creatures in the box.

In an alternative embodiment, the back wall 14 of the box 2 may be omitted or may be replaced by a transparent or translucent panel, so that the movements and behaviour of creatures in the box can be observed from inside the building to which the box 2 is fitted. Furthermore, by removing the back wall 14 of the box, access could be provided to a cavity behind the box 2, such that the box 2 operates as an opening into the cavity for use by creatures such as bats.

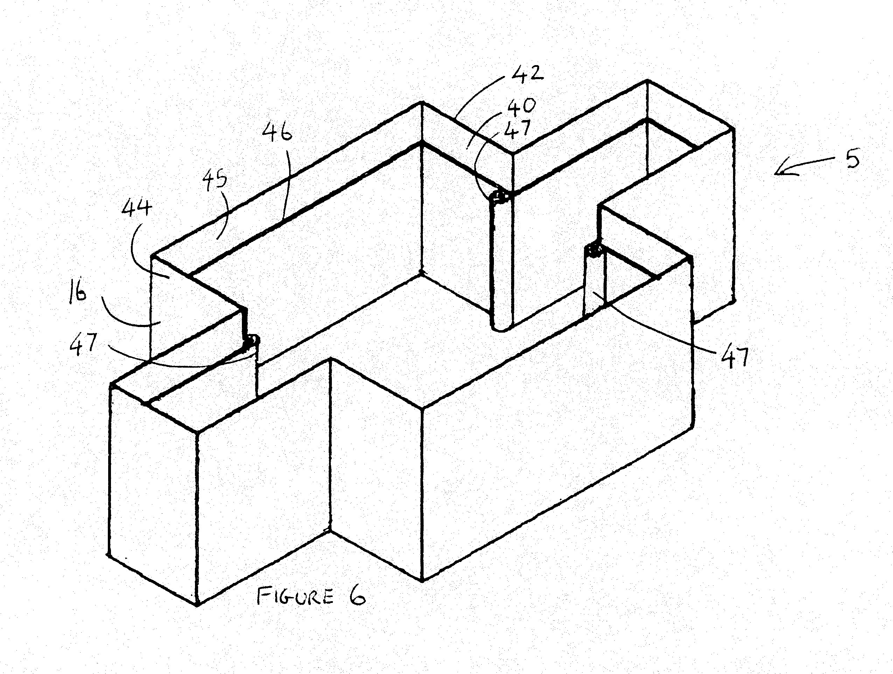

Figure 6 illustrates an embodiment in which a tapered lip 40 is provided around an open mouth 42 of the container portion 5. An outer face 44 of the lip may be continuous with the outer face of the peripheral wall 16, whereas an inner face 45 of the lip 40 may start at a shoulder 46 formed in the inner face of the peripheral wall 16 of the container portion 5, and may taper outwardly towards the mouth 42. Thus, the thickness of the lip 40 is less than the thickness of the peripheral wall 16 of the container portion 5 and reduces from a maximum thickness adjacent the shoulder 46 to a minimum thickness at the mouth 42 of the container portion 5.

In this embodiment, screw receiving sockets 47 are moulded onto the internal corners of the container portion 5. These sockets 47 may all finish flush with the shoulder 46, so that the lip 40 is not obstructed at any point.

In further embodiments, the lip 40 may be the same width as the peripheral wall 16 and/or may not be tapered. Also, the sockets 47 may not be set back to terminate level with the shoulder 46, but may extend along at least part of the lip 40.

In a further embodiment, the shoulder 46 may be discontinuous.

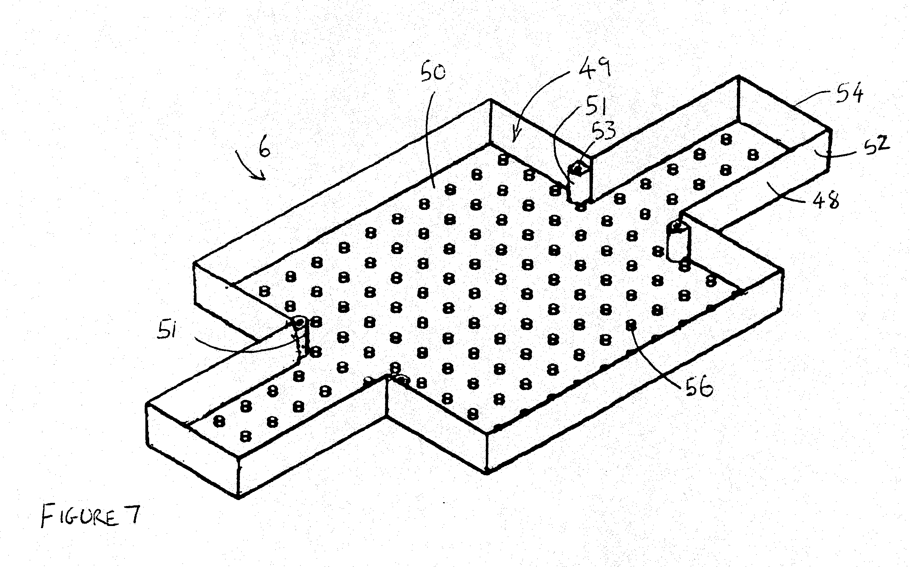

Figure 7 illustrates an embodiment of the cover or lid 6 which is in the form of a perforated tray 49, having a peripheral flange 48 which projects substantially at right angles to a perforated base 50 of the cover 6.

The peripheral flange 48 may have an outer surface 52 which is tapered outwardly towards a free edge 54 of the peripheral flange 48.

Fixation guides 51 having through bores 53 for suitable fixings such as screws or bolts (not shown) may be moulded or otherwise fixed to the corners of the cover 6.

The perforated base 50 comprises fixation holes 56, which are shown as having a circular cross section. In alternative embodiments, the fixation holes 56 may be any appropriate shape to admit a sufficient quantity of adhesive before it sets. For example the fixation holes 56 may be cross-shaped, L-shaped, T-shaped or polygonal in cross-section.

In a further embodiment, the cover 6 may comprise a framework, such as a mesh or a perforated panel, which is intended to improve the adhesion of a decorative surface covering (such a s slices of brick or building block) to the cover 6.

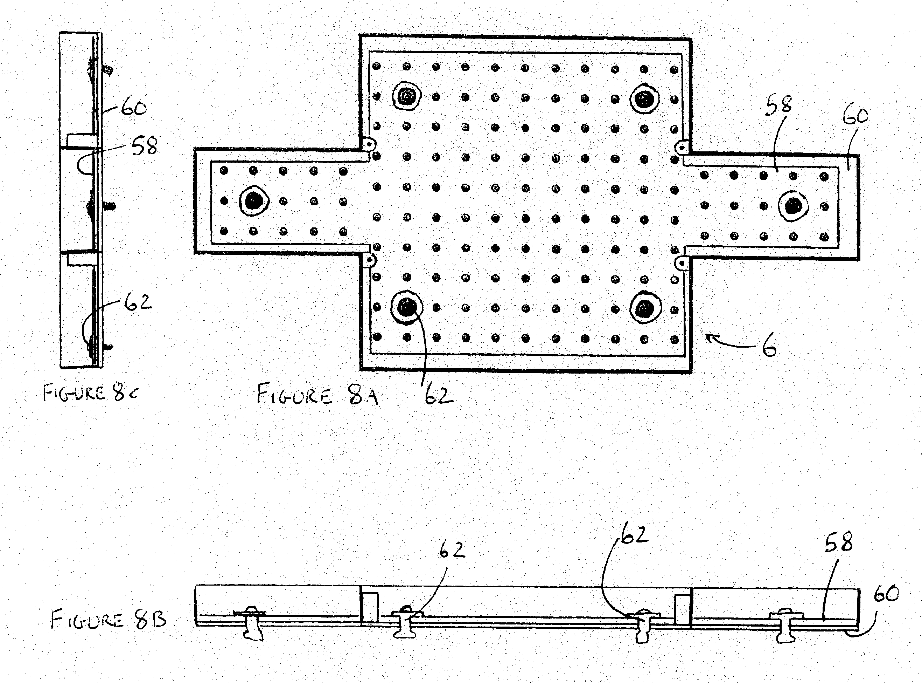

In figure 8A to 8C, the framework comprises a perforated panel 58 which is riveted or otherwise fixed to a tray shaped main body 60 of the cover. The framework may be secured in such a way that it remains spaced from the main body 60 of the cover. This may be achieved by placing washers or spacers (not shown) between the framework and the main body 60 of the cover 6. The washers or spacers may be held in place by fixings such as rivets 62.

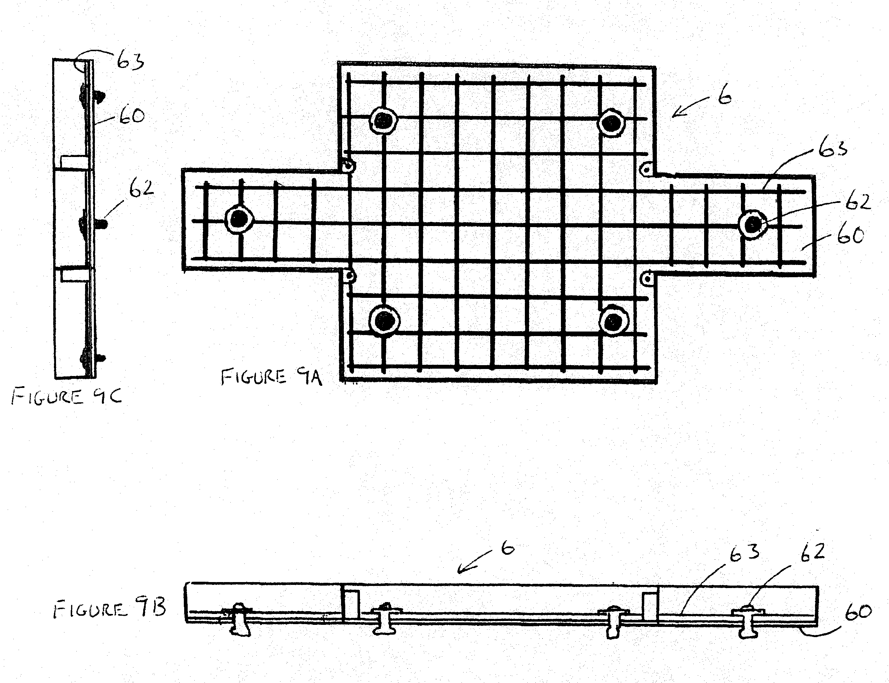

In the embodiment of figures 9A to 9C, the framework comprises a mesh panel 63 which may be fixed to the main body 60 of the cover 6 in the same way as the perforated panel 58 is fixed to the main body 60 of the cover 6 in the embodiment of Figure 8.

In use of a housing 4 as illustrated in Figures 7, 8A, 8B, 8C, 9A, 9B and 9C, decorative materials (such as slices of the building block from which the wall is formed) may be fixed to the cover 6 prior to assembly to the container portion 5. The decorative materials are fixed to the perforated tray 49, perforated panel 58 or mesh panel 63 with adhesive, and the size and shape of the fixation holes in the perforated tray 49, perforated panel 58 and mesh panel 63 are selected so that the adhesive (such as glue, grout, chemical bond or other settable material) used to fix the decorative covering seeps into the fixation holes 56 to improve the adhesive bond when the adhesive has set

In one embodiment, the fixation holes 56 are blind bores. In another embodiment the fixation holes 56 are sufficiently large to allow adhesive flow through them and run onto the opposite side of the perforated tray 49, perforated panel 58 or mesh panel 63. Thus, when the adhesive sets it forms an enlarged dome or blob of adhesive on the reverse side of the perforated tray 49, perforated panel 58 or mesh panel 63 which prevents the set adhesive being drawn back through the tray or panel if the adhesive bond fails in service, for example due to the cover 6 being dropped.

The fixation holes 56 may taper. If the fixation holes 56 taper outwardly to have a larger diameter on the opposite side of the perforated tray 49, perforated panel 58 or mesh panel 63 than on the side to which the decorative covering is fitted, when the adhesive sets it cannot be pulled back through the fixation holes 56 if the adhesive bond fails in service. A similar effect is achieved by forming the fixation holes 56 with a stepped bore, for example by drilling a first blind bore on the decorative cover side of the perforated tray or panel and then drilling a larger diameter bore to extend into the blind bore from the opposite side of the perforated tray or panel.

Once the decorative materials are fixed to the cover 6, it can be offered up to the open mouth 42 of the container portion 5 and is pushed into place until the inner face of the cover 6 abuts the shoulder 46 of the container portion 5, and the lip 40 lies against the flange 48.

Fixings such as screws are inserted through the bores 53 in the cover 6 into the aligned sockets 47 in the container portion 5. Alternatively, the cover 6 could be clipped in place using co-operating formations on the cover 6 and container portion 5. Where frequent access is necessary to the container portion 5, the cover 6 may be a simple friction fit in the container portion 5 or may be connected to it by a hinge along one edge. This arrangement would be particularly useful if the container portion 5 were fitted into the base of the wall and housed a trap or bait box.

If the container portion 5 has poison or insecticide, the cover 6 could be locked to the container portion 5 using a conventional lock mechanism, such as a simple keyed twist lock as formed on existing bait boxes.

With the cover 6 fixed in place, the housing can be built into a new wall as described in relation to the embodiments of Figures 1 to 6. Alternatively, some building blocks may be removed from an existing wall and the container portion 5 may then be inserted onto a mortar bed laid in the opening left by removal of the building blocks.

If the container portion 5 is provided with a lip 40, as described in the embodiment of Figure 6, it is laid into the wall so that the open mouth 42 of the container portion 5 sits substantially flush with a front face of the wall. The gaps between the container portion 5 and the surrounding building blocks and between the flange 48 and the decorative materials on the cover 6 are then grouted.

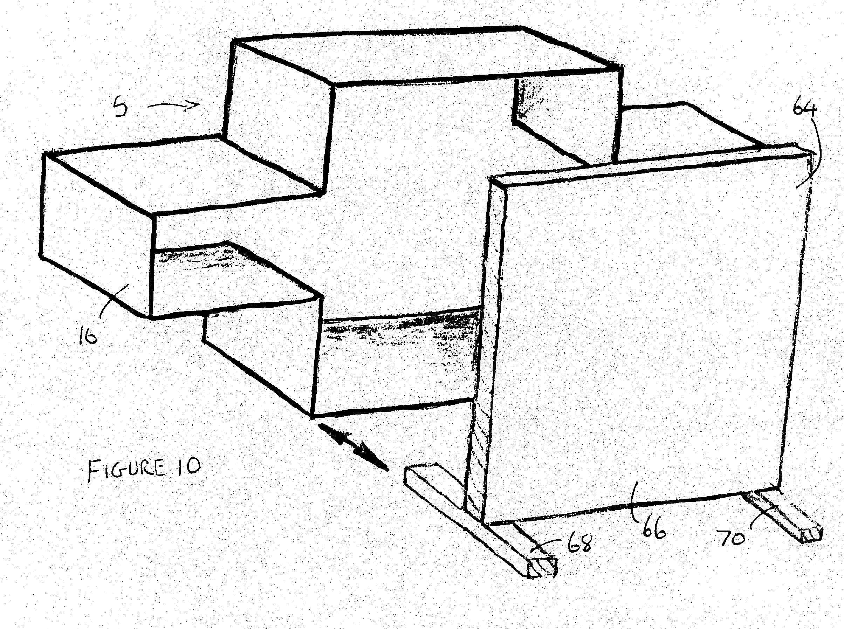

Figures 10 to 12 illustrate inserts 64 for the container portion 6 which are intended to provide a surface for birds insects or animals to hold on to. In the illustrated embodiment, the insert 64 comprises an oak plank or block acting as a grip element 66, which bats can hang from when they roost. The grip element 66 is provided with elongate feet 68, 70 which support the grip element 66, so that it is free standing in the container portion 5 and spaces the grip element 66 away from the peripheral wall 16 at the bottom of the container portion 5.

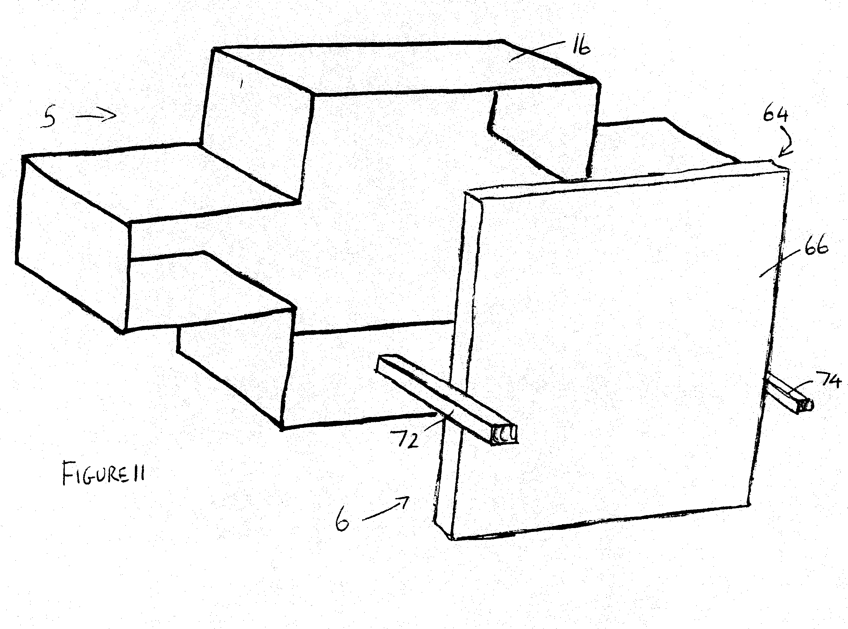

Figure 11 illustrate a further embodiment of insert 64 in which the elongate feet 68, 70 of Figures 10 and 11 are replaced by side support bars 72, 74 which rests on the substantially horizontally aligned portion of the inner face of peripheral wall 16 of the container portion 6. The side support bars 72, 74 support the grip element 66 and space it away from the peripheral wall 16 at the top and/or bottom of the container portion.

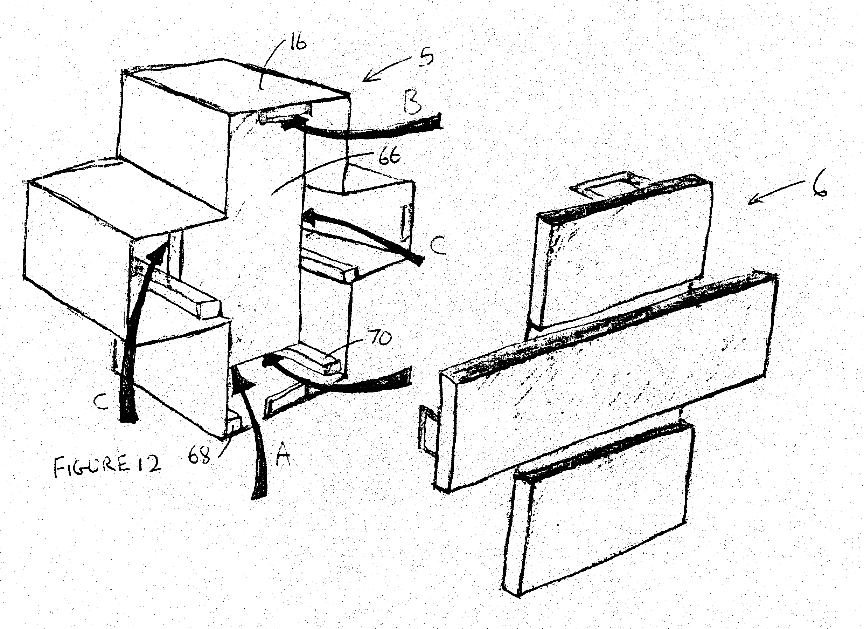

Figure 12 illustrates an embodiment using both side support bars 72, 74 and elongate support feet 68, 70 to provide increased stability for the grip element 66 when located in the container portion 6.

As a result of the deliberate spacing of the grip element 66 from the peripheral wall 16, bats can crawl along the path of arrows A under the grip element 66 to the far side of the grip element 66.

The height of the grip element 66 may be reduced to space the top of the grip element 66 from the peripheral wall 16 at the top of the container portion 5, so that bats can crawl along the path of arrow B over the top of the grip element 66 to access the far side of the grip element 66. It will be appreciated that if the container portion 5 is cross shaped (cruciate), as in the illustrated embodiments, access is provided to the far side of the grip element 66 around the sides of the grip element 66, so that the bats can follow the path of arrows C.

It will be appreciated that the insert 64 is easy to remove and install, for replacement or repair, simply by opening or removing the cover 6 and lifting the insert 64 out.

In a further embodiment, not illustrated, the insert 64 may comprise a hive support to assist honey bees in forming a hive in the container portion 6. Indeed, the insert 64 may comprise a plurality of separate honey comb support elements, such as might be provided in a commercial beehive to facilitate the farming of honey.

In a further embodiment, the insert 64 may be one of a number of replaceable and/or exchangeable "cartridges", which may be supplied as a kit. Each insert or cartridge can be used to adapt the housing 4 for different applications. For example, one cartridge could comprise the grip element 66, another could comprise a support for an insect hive, another could comprise a bait station, another could comprise a trap, such as a rat trap or other vermin trap, and another could comprise a surveillance unit comprising a camera and/or sound recording equipment and/or a proximity detector.

The insert 64 may be adapted to be closely received in the container portions and may be attached or attachable to the lid 6, so the insert 64 and lid 6 may be removed and replaced as a unit.

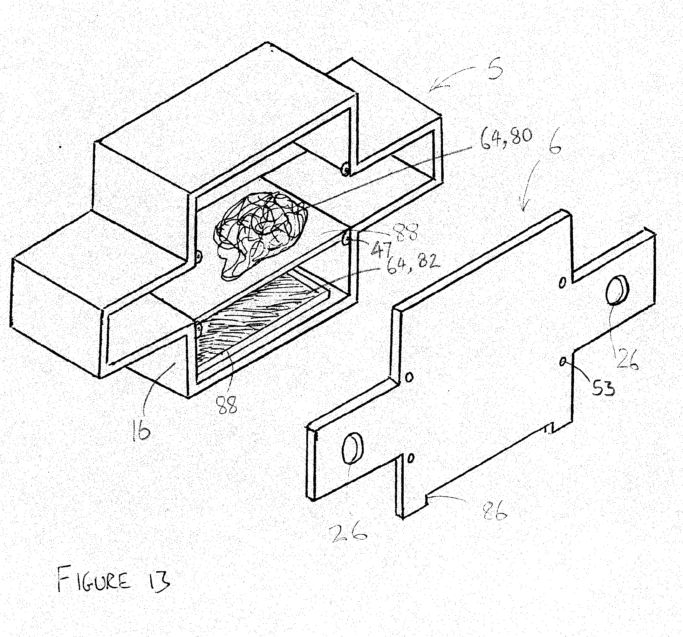

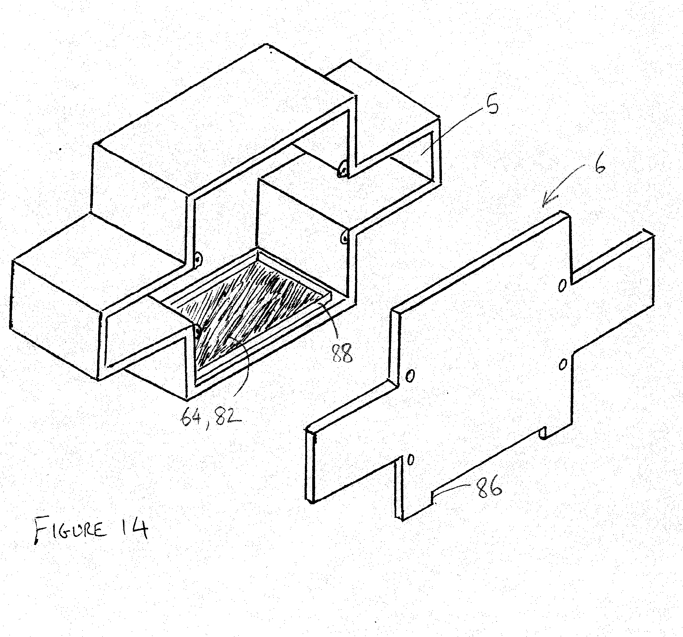

In the embodiments of Figures 13 to 17 the insert 64 comprises a bait station 80 or trap 82, the container portion 5 lid 6 and insert 64 together form a pest control unit which can be built into a structure. The container portion 5 may be built into a lower course of blocks of an interior or exterior wall. For example, if the container portion 5 is built into the interior wall of a restaurant, and is provided with a trap 82 or bait station 80 it could be used for catching or killing rodents or insects such as cockroaches. In the embodiment of Figures 13 and 14, the insert comprises a cockroach trap in the form of a sticky pad 84. When the cockroach walks onto the pad 84 it becomes stuck, and can be destroyed when the sticky trap 84 is cleaned off or replaced, or the sticky pad 84 may be impregnated with poison which kills the cockroach in situ.

The cover may be provided with an opening 26 or may be sized to provide a gap between the cover 6 and the container portion 5, so that creatures to be killed or trapped can access the container portion. In addition or instead, a wall of the container portion may be provided with an opening to allow creatures to enter the trap 82 or bait station 80 through a wall cavity or fissure in the wall. For example, if a rear wall of the container portion 5 is provided with an opening 26, the container portion 5 can be accessed by creatures which are present in the wall cavity. It will be appreciated that creatures such as rodents and cockroaches often seek out dark recesses in openings 26 in walls or gaps in structures, so a built-in trap 82 or bait station 80 with an appropriate opening from the wall cavity would be very attractive to the creatures for which the trap 82 or bait station 80 is intended.

As the trap 82 or bait station 80 is hidden from view within the structure to which the container portion 5 is fitted, it can be used in a hotel or restaurant or other public space without disturbing customers or other members of the public.

It can be off putting for a restaurant customer or hotel guest to see a bait station 80 or trap 82 in a restaurant or hotel, so being able to hide these essential structures from view, whilst enhancing their effectiveness is a very significant benefit of the present invention.

The lid 6 may hinged to the container portion 5 along a top edge, so that gravity alone keeps the lid 6 shut, or it may be hinged along a side or bottom edge and provided with a mechanism to hold the lid 6 closed. Alternatively, the lid 6 may be screwed or clipped to the container portion 5, or may be otherwise made completely removable from the container portion 5 for easy access to the container portion 5 and for replacement or substitution of one insert 64 with another of the same or a different type.

Figure 13 illustrates an alternative embodiment in which there are a plurality of inserts 64 in the container portion 5. The first insert comprises a rodent bait station 80 and the second insert comprises an insect trap 82 in the form of a sticky trap as described above. The insect trap 82 is located in the lower part of the container portion 5 and is accessed by an access opening comprising a slot 86 formed in a lower portion of the lid 6. The rodent bait station 80 comprises a tray 88 onto which a poisoned rodent bait is fixed, or onto which it may be poured. The tray 88 slides into the container portion 5 and is supported on raised rails (not shown) moulded into the peripheral wall 16 of the container portion 5. The rodent bait station 80 is accessed through openings 26 formed in the lid 6. It has been found that although insects access a trap or bait chamber more easily through a slot or gap formed at a lower portion of the cover, rodents easily access openings 26 formed at a higher level on the lid 6.

Figure 14 shows a simpler embodiment in which the inserts 64 comprise a sticky pad or tray, which is laid into a lower part of the container portion 5 and is accessed through a slot 86 formed in a lower portion of the lid 6.

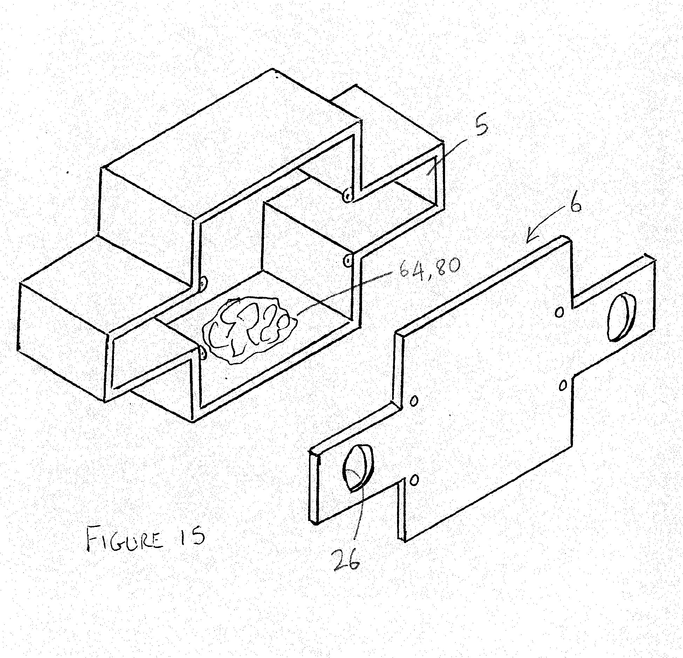

Figure 15 shows an alternative embodiment in which an insert 64 comprising a bait station 80 for rodents is inserted into a lower part of the container portion 5 and is accessed through two openings 26 formed in the lid 6.

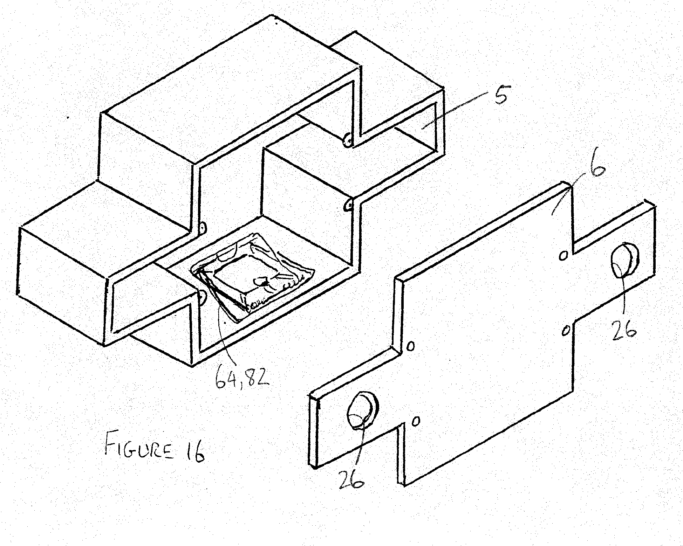

Figure 16 shows an alternative embodiment in which the insert 64 comprises a spring loaded rodent trap 82 which is located in a lower part of the container portion 5 and is accessed through openings 26 formed in the lid 6.

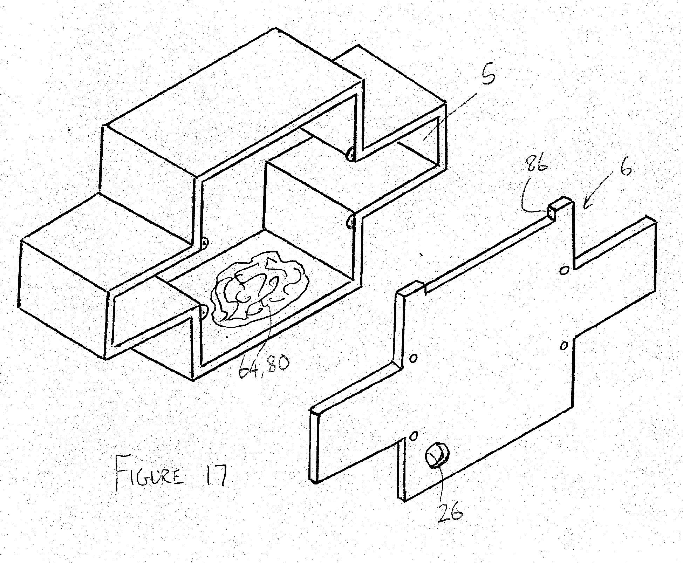

Figure 17 shows an embodiment having a reversible lid 6, which can be placed onto the container portion 5 in one of two possible orientations. In the first orientation, an opening 26 is located adjacent a lower part of the container portion 5 to allow access by rodents. In an alternative orientation, the cover is attached such that a slot 86 is aligned with a lower part of the container portion 5, to allow access by insects. It will be appreciated that the opening, slot or other access means into the container portion may be varied, so that alternative forms of reversible lid 6 may be provided depending on the type of creature which is to be accommodated in the container portion 5.

As in the previous embodiments, the lid 6 can be faced with a decorative covering, such as brick slivers, which are particularly appropriate for external applications. Alternatively, the lid 6 may be faced with a high grip surface, such as wire or mesh, so that plaster applied to the lid 6 can key into the wire or mesh. This is particularly suitable for an internal application, such as an internal wall in a restaurant. Alternatively, the lid can be left as bare plastic and may be coloured or moulded appropriately.

A back wall of the container portion 5 may be at least partially covered or lined with a plate, such as a thin metal plate, to stop rodents or insects gaining access to a wall cavity through the container portion 5. Alternatively, as mentioned above, holes may be provided in a back wall of the container portion deliberately to allow creatures such as rodents or insects to gain access to the container portion for trapping or poisoning.

The cover 6 can be attached to the container portion 5 using screws or locking cams with security heads to allow only permitted access to the inside of the container portion 5. In one possible embodiment, an oval locking catch could be provided on an inwardly directed face of the cover 6, which when rotated through 90 degrees, is oriented with its longitudinal axis substantially vertical, so that it can be inserted into the container portion 5. When the disc is then rotated through a further 90 degrees, such that its longitudinal axis lies substantially horizontal, it can engage with appropriate cut outs formed in lugs moulded onto the peripheral wall 16 of the container portion 5. In this way, the cover 6 can be locked in place. A locking disk of this kind may be rotated using a security key inserted through a key hole formed through the cover 6.

In its most basic form, the container portion 5 of any of the above described embodiments may be rectangular or square and could be no larger than a single building block of the wall into which it is inserted.

## Claims

1. Apparatus for nurturing or controlling creatures, the apparatus comprising a housing adapted to be built in to a structure, such as a wall floor or ceiling, and a plurality of removable and exchangeable inserts which are adapted to fit into the housing, each of the inserts having a different function.

2. The apparatus of claim 1 or 2, in which at least one of the inserts comprises a bait station.

3. The apparatus of any preceding claim, in which at least one of the inserts comprises a trap.

4. The apparatus of claim 1 or 2, in which at least one of the inserts is adapted to have a trap mounted on it.

5. The apparatus of any preceding claim, in which at least one of the inserts comprises a habitat enhancer which customises the housing for a particular bird, animal or insect.

6. The apparatus of claim 5, in which the habitat enhancer comprises a hive support for adapting the housing for use by bees or other hive insects.

7. The apparatus of claim 5, in which the habitat enhancer comprises a nest support frame for assisting birds in forming a nest.

8. The apparatus of any preceding claim, in which the housing comprises a container portion and a lid.

9. The apparatus of claim 8, in which the lid is provided with a peripheral flange which accommodates a decorative covering, the peripheral flange being closely received within the container portion.

10. The apparatus of claim 8 or 9, in which a free edge of the container portion comprises a mortar barrier which separates mortar on the lid from mortar applied to the wall.

11. The apparatus of any of claims 8 to 10, in which at least one of the inserts is connected to the lid.

12. The apparatus of claim 11, in which the at least one of the inserts is removably connected to the lid.

13. The apparatus of any of claims 8 to 12, in which the lid is hinged to the container portion.

14. The apparatus of any of claims 8 to 13, in which the lid and at least one of the inserts are provided with cooperating formations.

15. The apparatus of any preceding claim, in which the housing can only accommodate one of the inserts at a time.
